# EUROPÄISCHE PATENTSCHRIFT

(11) **EP 0 452 792 B1**
(45) Veröffentlichungstag und Bekanntmachung des Hinweises auf die Patenterteilung: **01.03.1995**
(21) Anmeldenummer: 91105668.7
(22) Anmeldetag: 10.04.1991
(51) Int. Cl.: A61B 17/60

(54) **Knochenchirurgischer Halter**
Fixator for bone surgery
Fixateur pour la chirurgie des os

(30) Priorität: 11.04.1990 DE 9004240 U
(43) Veröffentlichungstag der Anmeldung: 23.10.1991
(73) Patentinhaber: Waldemar Link (GmbH & Co.), D-22315 Hamburg (DE)
(72) Erfinder: Slot, G. H., Dr., NL-6522 Nijmegen (NL)
(74) Vertreter: Glawe, Delfs, Moll & Partner

(56) Entgegenhaltungen:
- WO-A-89/00028
- WO-A-91/01115
- BE-A- 496 725
- DE-A- 3 639 810
- GB-A- 2 041 139

## Beschreibung

Die Erfindung betrifft einen knochenchirurgischen Halter mit einer Gewindestange und einem Halteteil, der einen zwischen einem Mutternpaar an der Gewindestange zu befestigten Gabelkopf aufweist.

Halter dieser Art werden vornehmlich zur Distraktion und Kontraktion in der Wirbelsäulenchirurgie und bei der internen oder externen Fixation von Knochenfrakturen verwendet. Der Gabelkopf des Halteteils hat oftmals beträchtliche Kräfte aufzunehmen, die dazu zwingen, die Gabelfinger und auch den die Gabelfinger verbindenden Gabelsteg kräftig zu dimensionieren. Jedoch ist man andererseits bestrebt, die Abmessungen von zur zeitweiligen oder dauernden Implantation bestimmten Teilen möglichst gering zu halten. Dabei sollen die Muttern den Gabelkopf während einer gewissen Zeitspanne sicher an der Gewindestange halten, ohne daß sich die Position des Halters gegenüber der Gewindestange verändern kann. Zu diesem Zweck versucht die Erfindung zu erreichen, daß einerseits die Muttern einen hohen Lösewiderstand aufweisen und daß andererseits selbst dann, wenn eine Mutter sich gelockert haben sollte, der winkelfeste Verbund zwischen Halter und Gewindestange nicht gefährdet wird.

Diese Ziele werden nicht erreicht von bekannten Anordnungen (WO 89/00028 und DE-A 36 39 810), bei denen keine Maßnahmen zur Erhöhung des Lösewiderstands der Muttern getroffen sind und eine Winkelbeweglichkeit des Halters zur Gewindestange bei gelockerten Muttern gegeben ist.

WO-A-91/01115, ein Dokument das unter Art. 54(3) fällt, offenbart einen konischen Vorsprung an der Mutter und eine konische Vertiefung am Gabelkopf.

Die Erfindung erreicht das angegebene Ziel durch die Merkmale des Anspruchs 1.

Der höhere Lösewiderstand der erfindungsgemäßen Anordnung ergibt sich daraus, daß die Spannkraft der Muttern oder zumindest ein wesentlicher Teil derselben über die flächig konisch aneinander anliegenden Konusflächen der Vorsprünge bzw. Vertiefungen am Gabelkopf bzw. an der Mutter übertragen werden. Während bei einer Kraftübertragungsfläche, die lotrecht zur Kraftübertragungsrichtung verläuft, die mittlere Flächenpressung durch den Quotienten aus der übertragenden Kraft und der Kraftübertragungsfläche bestimmt wird, ist im Falle einer Kraftübertragung über eine Schrägfläche die Flächenpressung um den Faktor 1/sin alpha größer, wobei alpha der Winkel zwischen der Schrägfläche und der Kraftübertragungsrichtung ist. Bei einer Konusfläche gilt diese Beziehung gleichfalls angenähert, wobei alpha der Winkel zwischen einer Mantellinie und der Achse ist. Bei Wahl von alpha gleich 30 Grad wird auf diese Weise die Flächenpressung etwa verdoppelt. Entsprechend erhöht ist auch der Lösewiderstand. Daher besitzt die erfindungsgemäße Anordnung eine wesentlich höhere Sicherheit gegen unbeabsichtigtes Lösen der Muttern als bekannte Anordnungen. Dies gilt auch gegenüber derjenigen bekannten Anordnung (WO 89/00028), bei welcher die Vorsprünge am Gabelkopf und an den Muttern sphärisch ausgebildet sind, weil dort infolge der Verformungsmöglichkeit des Gabelkopfs nach innen von den sphärischen Kraftübertragungsflächen lediglich die Bereiche mit geringstem Neigungswinkel für die Kraftübertragung in Betracht kommen.

Ferner wird durch die erfindungsgemäßen Merkmale erreicht, daß die winkelfeste Verbindung zwischen dem Halteteil und der Gewindestange selbst dann nicht verloren geht, wenn eine Mutter sich geringfügig lockern sollte, weil dann immer noch der unschwenkbare Eingriff der Konusvorsprünge in des Gabelkopfs in die Konusvertiefungen der Muttern vorhanden ist.

Wie auch bei den bekannten Anordnungen mit sphärischen Zusammenwirken von Gabelkopf und Muttern, bietet die Erfindung den Vorteil, daß die in den Vertiefungen der Muttern gehaltenen Vorsprünge des Gabelkopfes diesen daran hindern, sich aufzubiegen. Auch wird die Fixierung des Halteteils während der Operation dadurch erleichtert, daß die Muttern schon dann, wenn sie sich dem Gabelkopf eine gewisse Distanz genähert haben, die Vorsprünge des Gabelkopfs umfassen und dadurch Eingriffssicherheit geben. Der Operateur kann anschließend die Muttern zum Justieren oder Aufrichten des Halteteils festziehen, ohne auf dem korrekten Sitz achten zu müssen; denn mit zunehmender Annäherung der Muttern an den Gabelkopf wird dieser aufgerichtet. Nach einem besonderen Merkmal der Erfindung wird die konische Vertiefung der Muttern von einem als dünner Rand auslaufenden Ringkragen gebildet. Dieses Merkmal gibt die Möglichkeit einer einfachen und sicheren Arretierung der Muttern, in dem der Rand im Zwischenraum zwischen den Fingern des Gabelkopfs, vorzugsweise benachbart der Innenkante eines Gabelfingers bzw. des darin gebildeten Vorsprungs, ein wenig eingebeult wird. Sollte sich die Mutter in Löserichtung zu drehen versuchen, schlägt die verformte Stelle des Rands an der Innenkante eines Gabelfingers oder Vorsprungs an, wodurch die weitere Drehung verhindert wird, solange die auf die Mutter wirkenden Drehkräfte eine bestimmte, in der praktischen Anwendung zu erwartende Schwelle nicht überschreiten.

Ferner kann nach der Erfindung vorgesehen sein, daß der dünne Rand des Ringkragens mit einer an der Gabel den Vorsprung umgebenden Fläche zusammenwirkt, die quer zur Kraftübertragungsrichtung (also in der Regel eben) verläuft. Ein solches Zusammenwirken kann auch dann stattfinden, wenn ein wesentlicher Teil der Kraft über die Konusflächen übertragen wird, weil diese sich unter dieser Kraft ein wenig nach innen verformen und damit dem Rand des Ringkragens die Möglichkeit geben, mit der genannten, dem Konusvorsprung umgebenden Fläche zusammenzuwirken. Dabei verformt sich der dünne Rand des Ringkragens ein wenig, wobei er im Bereich des Zwischenraums des Gabelkopfs ein wenig weiter in Kraftübertragungsrichtung vorragt als an der genannten Fläche. Daraus resultiert ein kräftiger Lösewiderstand selbst dann, wenn der Rand in den Gabelzwischenraum hinein nicht noch gesondert verformt wurde.

Um aber eine solche, besondere Verformung möglich zu machen, ist eine Mutter nach der Erfindung zweckmäßigerweise mit einem Widerlager für ein entsprechendes Verformungswerkzeug ausgerüstet.

Die Erfindung wird im folgenden näher unter Bezugnahme auf die Zeichnung erläutert, die vorteilhafte Ausführungsbeispiele veranschaulicht. Darin zeigen:
- Fig. 1: eine teilweise geschnittene Ansicht des Halters unter Verwendung einer Knochenschraube als Halteteil,
- Fig. 2: eine der Fig. 1 entsprechende Ansicht unter Verwendung eines Hakens als Halteteil,
- Fig. 3 und 4: zwei Ansichten des Hakens,
- Fig. 5 und 6: zwei Ansichten der Knochenschraube,
- Fig. 7 und 8: zwei Ansichten einer Mutter,
- Fig. 9: ein Detail im Schnitt
- Fig. 10: eine perspektivische Ansicht des Halters während der Montage und
- Fig. 11: ein Anwendungsbeispiel des Halters in der Wirbelsäulenchirurgie.

Der Halter besteht aus der Gewindestange 1, den Muttern 2, 3 und wenigstens einem Halteteil, nämlich der Knochenschraube 4 bzw. dem Haken 5. Die Knochenschraube 4 bzw. der Haken 5 sind mittels des Gabelkopfs 6 mit der Gewindestange 1 bzw. den Muttern 2, 3 verbunden. Der Gabelkopf 6 bildet zwei Gabelfinger 7, die durch den Gabelsteg 8 miteinander verbunden sind, der seinerseits mit dem Schraubenteil bzw. Hakenteil verbunden ist. Auf beiden Stirnseiten weist der Gabelkopf 6 einen an den Gabelfingern 7 und dem Steg 8 durchlaufenden Ringvorsprung 9 auf, dessen Außenkontur 10 konisch ist mit von den Fingern 7 aus geringer werdendem Außendurchmesser. Der Konuswinkel (gemessen zwischen zwei diametral gegenüberliegenden Mantellinien) beträgt in dem dargestellten Beispiel 60° und liegt vorzugsweise zwischen 30 und 120°, vorzugsweise zwischen 45 und 90°. Es ist nicht erforderlich, daß es sich um eine Kegelform im mathematischen Sinne handelt. Jedoch ist die dargestellte Form im allgemeinen zweckmäßig, weil sie auch bei verhältnismäßig großer Schräglage des Gabelkopfs gegenüber der Richtung der Gewindestange 1 und schon in einer gewissen Entfernung der Muttern vom Gabelkopf ein sicheres "Fangen" des Vorsprungs 9 durch die Muttern bei vergleichsweise geringem Raumbedarf gewährleistet.

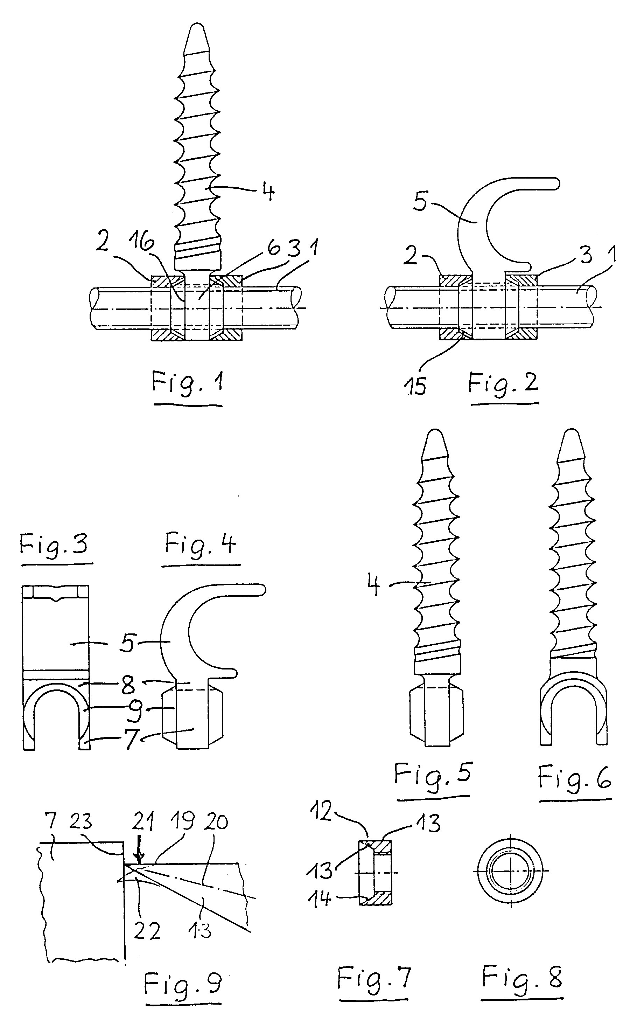

Die Muttern tragen in ihrem Umfangsbereich 11 geeignete Formationen für den Angriff eines Schlüssels, vorzugsweise Sechskantflächen. Im Bereich 12 sind sie zweckmäßigerweise zylindrisch zur Bildung des Ringkragens 13, dessen Innenkontur 14 im dargestellten Beispiel konisch ist und übereinstimmt mit der konischen Kontur 10 der Gabelkopfvorsprünge 9. Im montierten Zustand ergibt sich die in Fig. 1 und 2 dargestellte Konfiguration, wobei Spiel 15 zwischen den zusammenwirkenden Konusflächen angedeutet ist, das bei der praktischen Ausführung nicht vorhanden ist. Außerdem wirken die Muttern und der Gabelkopf über die ebene Stirnfläche 16 des Gabelkopfs zusammen, was zur Übertragung eines Biegemoments sowie zu der weiter unten erläuterten Mutternfixierung zweckmäßig sein kann. In anderen Fällen zieht man die alleinige Kraftübertragung über die zusammenwirkenden Konusflächen vor.

Fig. 10, die im wesentlichen mit der Ausführung gemäß Fig. 1 bis 8 übereinstimmt und sich davon nur durch die Beschränkung der Vorsprünge 9a auf den Bereich der Gabelkopffinger 7 unterscheidet, zeigt deutlich, daß die Erfindung es gestattet, daß der Halteteil 4 vor dem Zusammenziehen der Muttern 2, 3 gegenüber der Gewindestange 1 in hohem Maße unausgerichtet ist und dennoch durch das Zusammenwirken der Gabelkopfvorsprünge 9a mit dem Ringkragen 13 der Muttern richtig erfaßt und in die gewünschte, rechtwinklige Lage zur Gewindestange 1 gezwungen werden kann.

In dem Anwendungsbeispiel gemäß Fig. 11 sind zwei erfindungsgemäße Halter 17, 18 zur Distraktion von Wirbelkörpern eingesetzt, wobei sie durch einen Steg 18 miteinander zu einem H-Rahmen verbunden sind.

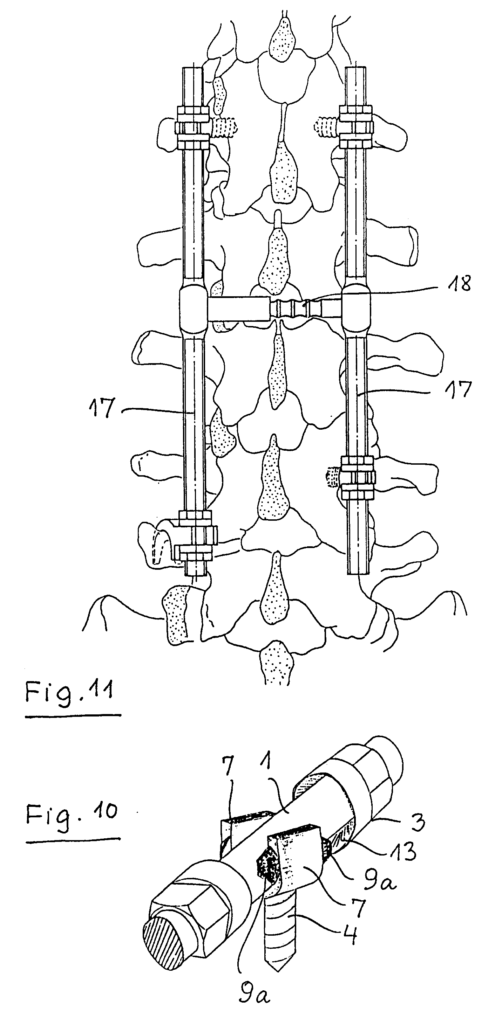

Wie man der vergrößerten Darstellung in Fig. 9 entnimmt, läuft der Ringkragen 14 in einem Rand 19 aus, der dünn ist und daher leicht mit geeigneten Werkzeugen verformt werden kann. Die Mittellinie 20 verläuft, vom Hauptkörper der Mutter her, nicht nur in axialer Richtung, sondern auch schräg nach außen. Wird nun mit Hilfe eines geeigneten Werkzeugs in Richtung des Pfeils 21 eine Kraft auf den Rand 19 ausgeübt, so wird er sich verbiegen, wie dies bei 22 angedeutet ist. Infolge der Schrägung der Mittellinie 20 erfolgt die Verformung nicht nur radial nach innen, sondern auch ein wenig in axialer Richtung. Nimmt man diese Verformung zwischen zwei Gabelkopffingern 7 vor, und zwar möglichst benachbart der Innenkante 23 dieser Finger, so erreicht man durch die Verformung 22 eine von den Kanten 23 der Gabelfinger 7 oder der Vorsprünge 9 eingefaßte Einbeulung, die die formschlüssige Sicherung der Mutter in der jeweiligen Stellung bewirkt. Zweckmäßig für die Fixierung ist, daß die Gabelfinger 7 den Rand 19 des Ringkragens 13 an derjenigen Stelle, an der der Rand 19 über die Innenkante der Gabelfinger hinwegläuft, überragen. Jedoch ist das nicht unbedingt erforderlich, da auch schon eine bloße Verformung radial nach innen bewirkt, daß eine mit einem Vorsprung zusammenwirkende Verformungsstelle entsteht, sofern der Vorsprung im Querschnitt dem Rand hinreichend benachbart ist.

## Patentansprüche

1. Knochenchirurgischer Halter mit einer Gewindestange (1) und einem Halteteil (4, 5), der einen zwischen einem Mutternpaar (2, 3) an der Gewindestange (1) zu befestigenden Gabelkopf (6) aufweist, wobei der Gabelkopf konzentrisch zu der vorgesehenen Stangenposition angeordnete Vorsprünge (9) und die Muttern (2, 3) Vertiefungen (14) aufweisen, die mit den Vorsprüngen (9) kraftübertragend zusammenwirken, wobei die Vorsprünge (9) und Vertiefungen (14) konisch ausgebildet sind.

2. Halter nach Anspruch 1, dadurch gekennzeichnet, daß die Vorsprünge (9) von einem an den Gabelfingern (7) und dem Gabelsteg (8) durchlaufenden Ringvorsprung gebildet sind.

3. Halter nach Anspruch 1 oder 2, dadurch gekennzeichnet, daß die konische Vertiefung (14) der Muttern von einem als dünner Rand (19) auslaufenden Ringkragen (13) gebildet ist.

4. Halter nach Anspruch 3, dadurch gekennzeichnet, daß dann, wenn die Mutter angezogen ist, der dünne Rand (19) des Ringkragens (13) verformt an einer Gegenfläche anliegt, die an der Gabel (6) den Vorsprung (9) umgibt.

5. Halter nach Anspruch 3 oder 4, dadurch gekennzeichnet, daß eine Mutter (2, 3) ein Widerlager für ein Werkzeug zum Verformen des Ringkragens (13) aufweist.

## Claims

1. A retaining means for bone surgery, with a threaded rod (1) and a retaining member (4, 5) which has a forked end (6) to be secured between a pair of nuts (2, 3) on the threaded rod (1), wherein the forked end has projections (9) arranged concentric to the intended rod position and the nuts (2, 3) have recesses (14) which co-operate with the projections (9) in a force-transmitting manner, wherein the projections (9) and recesses (14) are conically shaped.

2. A retaining means according to Claim 1, characterised in that the projections (9) are formed by an annular projection passing through the fork fingers (7) and the fork web (8).

3. A retaining means according to Claim 1 or 2, characterised in that the conical recess (14) of the nuts is formed by an annular collar (13) tapering into a thin edge (19).

4. A retaining means according to Claim 3, characterised in that when the nut is tightened the thin edge (19) of the annular collar (13) is applied deformed against a countersurface which surrounds the projection (9) on the fork (6).

5. A retaining means according to Claim 3 or 4, characterised in that a nut (2, 3) has an abutment for a tool for deforming the annular collar (13).

## Revendications

1. Appareil de contention pour chirurgie osseuse, comprenant une tige filetée (1) et un élément de fixation (4, 5) qui comporte une fourche (6) destinée à être fixée sur la tige filetée (1) entre deux écrous (2, 3), la fourche présentant des saillies (9) disposées concentriquement à la position prévue de la tige et les écrous (2, 3) présentant des creux (14) qui coopèrent avec les saillies (9) pour la transmission des efforts, les saillies (9) et les creux (14) étant réalisés sous forme conique.

2. Appareil de contention selon la revendication 1, caractérisé en ce que les saillies (9) sont constituées par une saillie annulaire qui fait le tour des doigts (7) et de la traverse (8) de la fourche.

3. Appareil de contention selon la revendication 1 ou 2, caractérisé en ce que le creux conique (14) des écrous est formé par un collet annulaire (13) qui se termine par un bord mince (19).

4. Appareil de contention selon la revendication 3, caractérisé en ce qu'au moment où l'écrou est serré, le bord mince (19) du collet annulaire (13) s'applique en se déformant sur une contre-surface qui entoure la saillie (9) sur la fourche (6).

5. Appareil de contention selon la revendication 3 ou 4, caractérise en ce qu'un écrou (2, 3) présente un appui pour un outil servant à produire la déformation du collet annulaire (13).
